Europäisches Patentamt

European Patent Office

Office européen des brevets

(11) Veröffentlichungsnummer: **0 021 325**
**B1**

(19)

(12) **EUROPÄISCHE PATENTSCHRIFT**

(45) Veröffentlichungstag der Patentschrift:
23.06.82

(21) Anmeldenummer: 80103372.1

(22) Anmeldetag: 18.06.80

(51) Int. Cl.³: **B 01 J 23/22**, B 01 J 27/18,
C 07 C 51/215, C 07 D 307/89,
B 01 J 37/02

(54) Vanadinpentoxid, Titandioxid, Phosphor, Rubidium und/oder Caesium und gegebenenfalls Zirkondioxid enthaltender Trägerkatalysator und Verwendung desselben.

(30) Priorität: 26.06.79 DE 2925682

(43) Veröffentlichungstag der Anmeldung:
07.01.81 Patentblatt 81/1

(45) Bekanntmachung des Hinweises auf die Patenterteilung:
23.06.82 Patentblatt 82/25

(84) Benannte Vertragsstaaten:
AT BE DE FR GB IT

(56) Entgegenhaltungen:
DE A 2 212 964
DE A 2 546 268
US A 3 926 846
US A 4 046 780

(73) Patentinhaber: BASF Aktiengesellschaft,
Carl-Bosch-Strasse 38, D-6700 Ludwigshafen (DE)

(72) Erfinder: Reuter, Peter, Dr., Richard-Wagner-Strasse 7,
D-6702 Bad Duerkheim 1 (DE)
Erfinder: Blechschmitt, Kurt, Finkenstrasse 15,
D-6707 Schifferstadt (DE)
Erfinder: Wirth, Friedrich, Dr., Schlossgasse 6,
D-6700 Ludwigshafen (DE)

## Vanadinpentoxid, Titandioxid, Phosphor, Rubidium und/oder Caesium und gegebenenfalls Zirkondioxid enthaltender Trägerkatalysator und Verwendung derselben

Diese Erfindung betrifft einen neuen Vanadinpentoxid, Titandioxid, Phosphor, Rubidium und/oder Caesium und gegebenenfalls Zirkondioxid enthaltenden Trägerkatalysator und seine Verwendung für die Herstellung von Phthalsäureanhydrid durch katalytische Gasphasenoxidation von o-Xylol oder Naphthalin.

Titandioxid und Vanadinpentoxid enthaltende Katalysatoren sind als Oxidationskatalysatoren für die Herstellung von Carbonsäuren oder Carbonsäureanhydriden aus aromatischen oder ungesättigten aliphatischen Kohlenwasserstoffen bekannt. Unter diesen Katalysatoren haben z. B. die in der FR-A 1 480 078 beschriebenen Trägerkatalysatoren, bei denen ein inertes Trägermaterial mit der katalytischen Masse überzogen ist, für die kontinuierliche Herstellung von Phthalsäureanhydrid (PSA) aus o-Xylol oder Naphthalin großtechnische Bedeutung erlangt.

Um die Dauerbelastbarkeit solcher Katalysatoren zu verbessern, gibt man der katalytischen Masse nach den Angaben der BE-A 737 587 Phosphorverbindungen hinzu. Die so erhaltenen phosphorhaltigen Katalysatoren können erst nach einigen Monaten mit der maximalen Kohlenwasserstoffmenge von bis zu 40 m/Nm³ Luft belastet werden. Um diesen Nachteil zu beheben, wurden in der DE-B 2 212 964 phosphorhaltige Katalysatoren vorgeschlagen, die in der äußeren Schicht der katalytischen Masse keinen oder nur bis zu 0,3 Gew.-% Phosphor und in der darunterliegenden katalytischen Masse 0,3 bis 6 Gew.-% Phosphor enthalten. Katalysatoren dieser Art können schon kurze Zeit nach der Inbetriebnahme mit der vollen Beladung von 40 g o-Xylol oder Naphthalin je Nm³ Luft belastet werden, ohne daß eine Schädigung des Katalysators eintritt.

Diese phosphorhaltigen Katalysatoren haben sich großtechnisch bewährt. Gegenüber phosphorfreien Katalysatoren besitzen sie außerdem den Vorteil, daß sie ohne den sonst üblichen Zusatz von Schwefeldioxid zum Gemisch aus o-Xylol bzw. Naphthalin und Luft betrieben werden können und damit die Umweltbelastung verringern. Sie haben jedoch den Nachteil, daß sie nur mit 40 g o-Xylol oder Naphthalin je Nm³ Luft belastet werden können. Wendet man mehr o-Xylol, z. B. bis 46 g je Nm³ Luft an, so bilden sich hotspots in einem engen Bereich innerhalb der Katalysatorfüllung mit Temperaturen von über 500°C. Bei so hohen Temperaturen tritt eine Schädigung des Katalysators ein, so daß die Katalysatorlebensdauer erheblich vermindert wird. Außerdem verringert sich die Ausbeute.

In den deutschen Offenlegungsschriften 2 421 406 und 2 436 009 werden Titandioxid und Vanadiumpentoxid enthaltende Katalysatoren beschrieben, die in der katalytischen Masse einen Gehalt von 0,01 bis 1,5 Gew.-% an Rubidium und/oder Caesium aufweisen. Diese Katalysatoren erlauben es, unter Beibehaltung einer hohen Ausbeute und Lebensdauer die Beladung an o-Xylol oder Naphthalin je Nm³ Luft auf 50 bzw. bis 150 g zu erhöhen. Mit ihnen lassen sich jedoch die vorteilhaften Eigenschaften der phosphorhaltigen Katalysatoren hinsichtlich der Dauerbelastbarkeit nicht erreichen. Außerdem ist von Nachteil, daß sie höchstens einige Monate ohne Schwefeldioxid-Zusatz zum Gemisch aus o-Xylol und Luft betrieben werden können und dann auch nur mit o-Xylolbeladungen unter 50 g je Nm³ Luft. Nach einigen Monaten Betriebszeit verschlechtert sich die Qualität des erhaltenen PSA, weshalb eine rasche Erhöhung der Salzbadtemperatur notwendig wird, die zwangsläufig zu einer Einbuße an Ausbeute führt. Um den Katalysator wirtschaftlich weiterbetreiben zu können, wird die Zugabe von Schwefeldioxid zum Synthesegemisch unumgänglich.

Die erforderliche Menge an Schwefeldioxid beträgt etwa 0,2 bis 1,5 Gew.-%, bezogen auf die o-Xylolmenge. Sie belastet die Umwelt und stellt einen nicht unerheblichen Kostenfaktor dar.

Als Maß für die Qualität des erhaltenen PSA dient dessen Gehalt an Phthalid, einem unteroxidierten Nebenprodukt, da dieses bei der Reinigung von PSA besondere Schwierigkeiten bereitet.

Einerseits ist es wegen der starken Umweltbelastung durch das in die Atmosphäre gelangende Schwefeldioxid erwünscht, die Katalysatoren ohne $SO_2$-Zusatz zum Gasgemisch zu betreiben, andererseits ist man bestrebt, möglichst hohe Beladungen an o-Xylol bzw. Naphthalin je Nm³ Luft zu erreichen, weil man dann mit einer geringeren Menge an zu kompromierender Luft auskommt, was zu erheblichen Einsparungen an Stromkosten oder Kosten für Dampf führt, wodurch sich die Wirtschaftlichkeit des Verfahrens stark verbessern läßt.

Beide Maßnahmen, die $SO_2$-freie Betriebsweise und die Betriebsweise mit hohen o-Xylolbeladungen der Luft schließlich sich bei den bekannten Katalysatoren gegenseitig aus. Es bestand deshalb die Aufgabe, einen Katalysator zu finden, der sich sowohl ohne $SO_2$-Zugabe als auch mit hohen Beladungen oder Luft mit o-Xylol betreiben läßt und dabei die geforderten vorteilhaften Ergebnisse hinsichtlich der PSA-Ausbeute und Reinheit liefert.

Die naheliegende Möglichkeit, Katalysatoren zu verwenden, die sowohl Phosphor als auch Rubidium und/oder Caesium in der katalytischen Masse enthalten, führte nicht zu dem erwünschten Ziel. Mit diesen Katalysatoren können zwar hohe o-Xylolbeladungen der Luft von über 40 g je Nm³ erreicht werden, jedoch bringen sie weniger Ausbeute als wenn man allein die phosphorhaltigen Kontakte oder allein die rubidium- und/oder caesiumhaltigen Kontakte verwendet. Auch eine Betriebsweise ohne $SO_2$-Zusatz zum Synthesegemisch ist mit Rubidium und Caesium enthaltenden Katalysatoren nur über wenige Monate möglich. Dies gilt auch für das in der DE-A 2 546 268 beschriebene Verfahren, beidem man zwei verschiedene Katalysatoren so in dem Reaktionsrohr anordnet, daß das gasförmige Ausgangsgemisch zuerst mit einem Rubidium enthaltenen Katalysator und dann mit einem Phosphor enthaltenen Katalysator in Berührung kommt.

Es wurde nun gefunden, daß ein Vanadinpentoxid, Titandioxid, Phosphor, Rubidium und/oder Caesium und gegebenenfalls Zirkoniumdioxid enthaltender Trägerkatalysator, der durch Aufbringen einer entsprechenden Lösung oder Suspension der Komponenten auf einen inerten Träger in zwei Schichten so hergestellt wurde, daß der Katalysator nach der Trocknung in einer Schichtdicke von 0,02 bis 2 mm mit der katalytisch wirksamen Masse beschichtet ist, die gewünschten Vorzüge aufweist, wenn man als katalytisch wirksame Masse eine solche verwendet, die 1 bis 40 Gew.-% $V_2O_5$, 60 bis 99 Gew.-% $TiO_2$ und/oder $ZrO_2$ und bezogen auf die Gesamtmenge an $TiO_2$, $ZrO_2$ und $V_2O_5$ bis zu 2 Gew.-% Phosphor und bis zu 1,5 Gew.-% Rubidium und/oder Caesium enthält und das Aufbringen der Masse in der Weise durchgeführt wird, daß der vorerhitzte Träger zuerst mit einer Schicht der katalytisch wirksamen Masse beaufschlagt wird, die über 0,2 bis 2 Gew.-% Phosphor aber kein Rubidium und/oder Caesium enthält und der so mit einer ersten, inneren Schicht beschichtete Träger mit einer zweiten, äußeren Schicht der katalytisch wirksamen Masse beaufschlagt wird, die 0 bis 0,2 Gew.-% Phosphor und 0,02 bis 1,5 Gew.-% Rubidium und/oder Caesium enthält, wobei man das Aufbringen so vornimmt, daß die katalytisch wirksame Masse der phosphorfreien oder phosphorärmeren äußeren Schicht des Trägerkatalysators 10 bis 90 Gew.-% der gesamten katalytisch wirksamen Masse beträgt.

Die katalytische Masse kann außer den genannten Bestandteilen noch kleine Mengen, z. B. bis zu 10 Gew.-% eines Oxids der Metalle, Niob, Zinn, Silicium, Antimon, Hafnium, Molybdän oder Wolfram enthalten.

$TiO_2$ liegt in den neuen Katalysatoren vorteilhaft als Anatas mit einer inneren Oberfläche von 5 bis 30, insbesondere 5 bis 20 m²/g vor. Auch die innere Oberfläche des Zirkondioxids soll möglichst zwischen 2 und 25, insbesondere 2 bis 20 m²/g liegen. Diese Oxide werden in feinverteilter Form angewandt.

Als wesentliches Merkmal der Erfindung enthalten die neuen Katalysatoren in der äußeren Schicht der katalytisch aktiven Masse keinen oder nur bis zu 0,2 Gew.-% Phosphor und 0,02 bis 1,5 Gew.-%, vorzugsweise 0,05 bis 0,5 Gew.-% Rubidium und/oder Caesium und in der darunterliegenden katalytisch wirksamen rubidium- und caesiumfreien Masse 0,2 bis 2 Gew.-%, vorzugsweise 0,3 bis 1,2 Gew.-% Phosphor, jeweils bezogen auf die Gesamtmenge $V_2O_5$, $TiO_2$ und gegebenenfalls $ZrO_2$.

Die äußere Schicht des Trägerkatalysators beträgt vorzugsweise höchstens die Hälfte der gesamten katalytisch wirksamen Masse.

Die Katalysatoren dieser Erfindung sind Trägerkatalysatoren, die aus einem inerten Träger und der auf dem Träger in dünner Schicht aufgebrachten katalytisch aktiven Masse bestehen. Sie enthalten als inerten Träger ein möglichst porenarmes Material, das vorzugsweise eine innere Oberfläche von 0 bis 3 m³/g aufweist, wie Quarz, Porzellan, geschmolzenes Aluminium. Siliciumcarbid und geschmolzene oder gesinterte Silikate. Die Träger liegen vorteilhaft in Form von Körnern, Pillen, Kugeln oder anderen Formkörpern, insbesondere aber in Form von Ringen vor.

Die gesamte aktive Masse befindet sich auf dem Träger zweckmäßig in einer Menge von 2 bis 140, vorzugsweise 2 bis 50 Gew.-%, bezogen auf den Trägerkatalysator.

Der Vanadiumpentoxidgehalt des Trägerkatalysators beträgt vorzugsweise 0,05 bis 3 Gew.-%.

Die Beschichtung des Trägers mit der katalytischen Masse wird zweckmäßig so durchgeführt, daß man eine Lösung oder Suspension einer Vanadiumverbindung und einer Phosphorverbindung und gegebenenfalls einer Rubidium- oder Caesiumverbindung mit feinverteiltem Anatas und gegebenenfalls Zirkondioxid anpastet und die Paste in einer Dragiertrommel auf den 200 bis 450°C vorerhitzten Träger aufsprüht. Ein nachträgliches Erhitzen des Katalysators auf 400 bis 500°C kann dabei von Vorteil sein. Als Vanadinverbindung kommen z. B. Vanadyloxalat, Vanadylformiat, Vanadyltartrat, Ammoniumvanadat oder Vanadinpentoxid und als Phosphorverbindung z. B. Alkali- und Ammoniumphosphate, die entsprechenden Meta- oder Pyrophosphate und Erdalkaliphosphate oder Phosphorsäure oder ein Ester der Phosphorsäure in Betracht. Als Rubidium- und Caesiumverbindungen verwendet man z. B. die Oxide oder andere sauerstoffhaltige Verbindungen, die bei erhöhter Temperatur in die Oxide übergehen, wie Carbonate, Acetate und Nitrate. Die einzelnen Schichten erzeugt man durch separates Aufbringen der jeweiligen Massen in der gewünschten Reihenfolge.

Die neuen Katalysatoren eignen sich z. B. für die Herstellung von Phthalsäureanhydrid durch Oxidation von o-Xylol oder Naphthalin, für die Herstellung von Maleinsäureanhydrid durch Oxidation von Benzol oder ungesättigten aliphatischen $C_4$-Kohlenwasserstoffen, zur Herstellung von Pyromellithsäureanhydrid durch Oxidation aus Durol oder anderen 1,2,4,5-Tetraalkylbenzolen, zur Herstellung von Naphthalsäure aus Acenaphthen, zur Herstellung von Chinonen durch Oxidation von Naphthalin zu Naphthochinon, oder durch Oxidation von Anthracen, substituierten Indanen oder von Diphenylmethanverbindungen, z. B. von 2-Methyldiphenylmethan zu Anthrachinon mit Luft oder einem sauerstoffhaltigen Gas.

Von besonderem technischen Interesse ist die Verwendung des neuen Katalysators für die Herstellung von Phthalsäureanhydrid durch Luftoxidation von o-Xylol oder Naphthalin. Dabei kann der Katalysator auf an sich bekannte Weise, z. B. wie in der FR-A 1 480 078 beschrieben, in Betrieb genommen werden.

Man kann den neuen Katalysator hierbei auch vorteilhaft so einsetzen, daß er in dem Reaktorrohr nur die ersten 25 bis 70% Volumenprozent in Strömungsrichtung des Gemisches aus dem

3

Kohlenwasserstoff und dem sauerstoffhaltigen Trägergas einnimmt. Die restlichen 30 bis 75 Volumenprozent des Katalysatorvolumens werden bei dieser Arbeitsweise mit einem üblichen Katalysator beschickt, z. B. mit einem in der DE-B 1 769 998 beschriebenen Katalysator, der z. B. aus einem inerten Träger und einer katalytisch wirksamen Masse besteht, die sich aus 66 bis 99 Gew.-% Titandioxid und 1 bis 40 Gew.-% Vanadinpentoxid zusammensetzt und die, bezogen auf Titandioxid, 0,02 bis 0,8 Gew.-% Phosphor in Form einer Phosphorverbindung enthält.

## Beispiel 1

### a) Herstellung des Katalysators

In einer Dragiertrommel werden 1200 g Steatitringe mit einem äußeren Durchmesser von 8 mm und einer Länge von 6 mm auf 260°C erhitzt und mit einer wäßrigen Suspension, bestehend aus 250 g Anatas mit der inneren Oberfläche von 11 m²/g, 81 g Formamid, 500 g Wasser, 35,9 g Vanadyloxalat (entspricht 16 g $V_2O_5$) und 4,44 g Ammoniumdihydrogenphosphat (entspricht 3,66 g $PO_4^{3-}$) besprüht, bis der Anteil der aktiven Masse am Trägerkatalysator 5 Gew.-% beträgt. Der Überzug mit aktiver Masse enthält 94 Gew.-% $TiO_2$, 6 Gew.-% $V_2O_5$ und 0,45 Gew.-% Phosphor als Phosphat, bezogen auf die Gesamtmenge Anatas und Vanadinpentoxid.

Anschließend wird eine wäßrige Suspension, bestehend aus 250 g Anatas mit der inneren Oberfläche von 11 m²/g, 81 g Formamid, 500 g Wasser und 35,9 g Vanadyloxalat (entspricht 16 g $V_2O_5$) und 0,54 g Rubidiumcarbonat aufgesprüht, bis der gesamte Anteil der aktiven Massen am Trägerkatalysator 10 Gew.-% beträgt. Der zweite Überzug enthält 94 Gew.-% $TiO_2$, 6 Gew.-% $V_2O_5$ und 0,15 Gew.-% Rubidium, bezogen auf die Gesamtmenge Anatas und Vanadinpentoxid.

### b) Oxidation

1180 g des Katalysators werden in ein 3,25 m langes Eisenrohr mit einer lichten Weite von 25 mm eingefüllt. Das Esienrohr ist zur Temperaturregelung von einer Salzschmelze umgeben. Durch das Rohr werden stündlich von oben nach unten 4,5 Nm³ Luft mit Beladungen an 97gewichtsprozentigem o-Xylol bis etwa 60 g geleitet. Dabei werden die in folgender Tabelle zusammengefaßten Ergebnisse erhalten (Ausbeute bedeutet das erhaltene Phthalsäureanhydrid in Gewichtsprozent, bezogen auf 100%iges o-Xylol):

| Versuchs-dauer Monate | Beladung der Luft g o-Xylol/Nm³ | Salzbad-temperatur (°C) | Ausbeute Gew.-% | Phthalidgehalt im Roh-PSA (%) |
|---|---|---|---|---|
|  | 36,0 | 380 | 112,1 | Spuren |
| 0,5 | 50,3 | 372 | 113,6 | Spuren |
| 1,0 | 60,8 | 364 | 113,6 | 0,001 |
| 9,0 | 62,3 | 364 | 114,1 | 0,002 |

Nach einer Versuchsdauer von 9 Monaten ist kein Nachlassen der Katalysatoraktivität zu beobachten, wie der praktisch unveränderte Gehalt an Phthalid im erhaltenen Phthalsäureanhydrid zeigt. Ein Zusatz von Schwefeldioxid zum Synthesegas ist nicht notwendig.

## Beispiel 2

### a) Herstellung des Katalysators I

Der Katalysator wird wie in Beispiel 1 beschrieben hergestellt.

### b) Herstellung des Katalysators II

600 g Steatit-Ringe mit einem äußeren Durchmesser von 8 mm und einer Länge von 6 mm werden in einer Dragiertrommel auf 260°C erhitzt und mit einer Suspension, bestehend aus 200 g Anatas mit einer inneren Oberfläche von 11 m²/g, 36,6 g Vanadyloxalat (Vanadiumgehalt entspricht 41% $V_2O_5$),

200 g Wasser, 50 g Formamid und 2,44 g Ammoniumhydrogenphosphat besprüht, bis das Gewicht der aufgetragenen katalytischen Masse 10% vom Gesamtgewicht des Katalysators ausmacht. Die so aufgebrachte katalytische Schicht besteht aus 0,3 Gew.-% Phosphor, 7,0 Gew.-% Vanadinpentoxid und 92,7 Gew.-% Titandioxid.

### c) Oxidation

680 g des Katalysators II und anschließend 500 g des Katalysators I werden in ein 3,25 m langes Eisenrohr mit einer lichten Weite von 25 mm eingefüllt. Die Versuche werden wie in Beispiel 1 beschrieben durchgeführt. Dabei werden folgende Ergebnisse erhalten:

| Versuchs-dauer Monate | Beladung der Luft g o-Xylol/Nm$^3$ | Salzbad-temperatur (°C) | Ausbeute Gew.-% | Phthalidgehalt im Roh-PSA (%) |
|---|---|---|---|---|
| | 35 | 379 | 111,8 | Spuren |
| 0,5 | 49 | 372 | 113,2 | Spuren |
| 1,0 | 62,1 | 362 | 113,2 | Spuren |
| 10,0 | 61,8 | 360 | 113,9 | Spuren |

Nach 10 Monaten Versuchsdauer kann der Katalysator noch ohne Schwefeldioxid-Zusatz zum Synthesegas betrieben werden. Der Phthalidgehalt im erhaltenen Phthalsäureanhydrid ist unverändert.

Ein Zusatz von Schwefeldioxid zum Synthesegas ist nicht notwendig.

## Patentansprüche

1. Vanadinpentoxid, Titandioxid, Phosphor, Rubidium und/oder Caesium und gegebenenfalls Zirkoniumdioxid enthaltender Trägerkatalysator durch Aufbringen einer entsprechenden Lösung oder Suspension der Komponenten auf einen inerten Träger in zwei Schichten mit der Maßgabe, daß der Katalysator nach der Trocknung in einer Schichtdicke von 0,02 bis 2 mm mit der katalytisch wirksamen Menge beschichtet ist, dadurch gekennzeichnet, daß man als katalytisch wirksame Masse eine solche verwendet, die 1 bis 40 Gew.-% $V_2O_5$, 60 bis 99 Gew.-% $TiO_2$ und/oder $ZrO_2$ und, bezogen auf die Gesamtmenge an $TiO_2$, $ZrO_2$ und $V_2O_5$, bis zu 2 Gew.-% Phosphor und bis zu 1,5 Gew.-% Rubidium und/oder Caesium enthält, wobei der Phosphor in Form von Phosphorverbindungen und Rubidium und Caesium in Form ihrer Oxide oder anderer sauerstoffhaltiger Verbindungen, die bei erhöhter Temperatur in die Oxide übergehen, vorliegen, und das Aufbringen der Masse in der Weise durchgeführt wird, daß der vorerhitzte Träger zuerst mit einer Schicht der katalytisch wirksamen Masse beaufschlagt wird, die über 0,2 bis 2 Gew.-% Phosphor, aber kein Rubidium und/oder Caesium, enthält und der so mit einer ersten, inneren Schicht beschichtete Träger mit einer zweiten, äußeren Schicht der katalytisch wirksamen Masse beaufschlagt wird, die 0 bis 0,2 Gew.-% Phosphor und 0,02 bis 1,5 Gew.-% Rubidium und/oder Caesium enthält, wobei man das Aufbringen so vornimmt, daß die katalytisch wirksame Masse der phosphorfreien oder phosphorärmeren äußeren Schicht des Trägerkatalysators 10 bis 90 Gew.-% der gesamten katalytisch wirksamen Masse beträgt.

2. Verwendung des Trägerkatalysators nach Anspruch 1 für die Herstellung von Phthalsäureanhydrid durch Luftoxidation von o-Xylol oder Naphthalin.

## Claims

1. A supported catalyst which contains vanadium pentoxide, titanium, dioxide, phosphorus, rubidium and/or cesium, with or without zirconium dioxide, obtained by applying an appropriate solution or suspension of the components in two layers onto an inert carrier, so that, after drying, the catalyst acrries an 0.02 to 2 mm thick coating of the catalytic material, characterized in that the catlytic material used contains from 1 to 40% by weight of $V_2O_5$, from 60 to 99% by weight of $TiO_2$ and/or $ZrO_2$ and, based on the total amount of $TiO_2$, $ZrO_2$ and $V_2O_5$, up to 2% by weight of phosphorus and to 1.5% by weight of rubidium and/or cesium, the phosphorus being present in the form of phosphorus compounds, and rubidium and cesium being present in the form of their oxides or other oxygen-containing compounds which are converted to the oxides at elevated temperature, and the application of the material is effected by first providing the preheated carrier with a layer of the catalytic material which contains more than 0.2 and up to 2% by weight of phosphorus but no rubidium nad/or cesium and then providing the carrier, thus coated with a first, inner layer, with a second, outer layer of the catalytic material which contains from 0 to 0.2% by weight of phosphorus and from 0.02 to 1.5% by weight of rubidium and/or cesium, the coating being applied in such a way that the catalytic material of the outer layer — which is free from phosphorus or has the lower phosphorus content — of the supported catalyst accounts for from 10 to 90% by weight of the total catalytic material.

2. Use of a supported catalyst as claimed in claim 1 for the preparation of phthalic anhydride by oxidizing o-xylene or naphthalene with air.

## Revendications

1. Catalyseur sur support contenant du pentoxyde de vanadium, du bioxyde de titane, du phosphore, du rubidium et/ou du caesium et le cas échéant du dioxyde de zirconium, revêtu par application d'une solution ou suspension correspondante des composants sur un support inerte en deux couches dans des conditions telles que le catalyseur, après séchage est revêtu de la masse catalytique active avec une épaisseur de couche de 0,02 à 2 mm, caractérisé en ce que l'on utilise en tant que masse catalytique active une masse contenant de 1 à 40% en poids de $V_2O_5$, de 60 à 99% en poids de $TiO_2$ et/ou de $ZrO_2$ et, par rapport à la quantité totale de $TiO_2$, $ZrO_2$ et $V_2O_5$, jusqu'à 2% en poids de phosphore et jusqu'à 1,5% en poids de rubidium et/ou de caesium, le phosphore étant à l'état de composés du phosphore et le rubidium et le caesium à l'état d'oxydes ou d'autres composés oxygénés qui se transforment en les oxydes à température élevée, et l'application de la masse étant réalisée dans les conditions siuvantes : sur le support chauffé auf préalable on applique d'abord une couche de la masse catalytique active qui contient plus de 0,2% en poids et jusqu'à 2% en poids de phosphore mais pas de rubidium et/ou de caesium, et sur le support ainsi revêtu d'une première couche interne, on applique une deuxième couche externe de la masse catalytique active qui contient de 0 à 0,2% en poids de phosphore et de 0,02 à 1,5% en poids de rubidium et/ou de caesium, l'application étant réalisée de manière que la masse catalytique active de la couche extérieure, exempte de phosphore ou plus pauvre en phosphore, du catalyseur sur support représente de 10 à 90% du poids de la masse catalytique active totale.

2. Utilisation du catalyseur sur support selon la revendication 1 pour la préparation de l'anhydride phthalique par oxydation à l'air de l'o-xylène ou du naphthalène.